(19) Europäisches Patentamt
European Patent Office
Office européen des brevets

(11) Publication number: **0 332 038 A2**

# (12) EUROPEAN PATENT APPLICATION

(21) Application number: 89103591.7

(22) Date of filing: 01.03.89

(51) Int. Cl.[4]: **C07C 87/58 , //C08G18/00**

(30) Priority: 07.03.88 US 164602

(43) Date of publication of application: 13.09.89 Bulletin 89/37

(84) Designated Contracting States: AT BE CH DE FR GB IT LI NL SE

(71) Applicant: **AIR PRODUCTS AND CHEMICALS, INC.**
P.O. Box 538
Allentown, Pennsylvania 18105(US)

(72) Inventor: **Burgoyne, William Franklin, Jr.**
1090C Cold Stream Circle
Emmaus, PA 18049(US)
Inventor: **Casey, Jeremiah Patrick**
2665 Arbor Circle
Emmaus, PA 18049(US)
Inventor: **Dixon, Dale David**
2216 Parklake Pointe
Venice, FL 33293(US)
Inventor: **Fowlkes, Robert Lee**
220 Cedar Street
Milton Florida 32570(US)

(74) Representative: **Kador & Partner**
Corneliusstrasse 15
D-8000 München 5(DE)

## (54) Tert-alkyl-phenylenediamines.

(57) This invention relates to a class of tert-alkyl-para-phenylenediamines having alkyl groups ortho to an amine group. More particularly, the aromatic diamines are represented by the formulas:

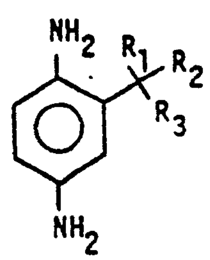

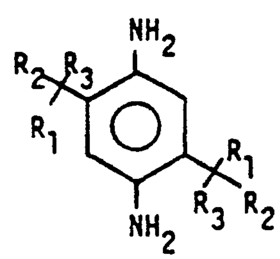

wherein $R_1$, $R_2$ and $R_3$ are $C_{1-3}$ alkyl groups or $R_2$ and $R_3$ are combined to form a $C_{5-6}$ membered ring.

The above described alkylated para-phenylenediamines have been found to be well suited for use as a chain extender in forming polyurethane/urea elastomer systems and provide enhanced benefits in terms of delayed reactivity for facilitating the processing of the elastomer systems, and high tensile strength, thermal stability and tear resistance in the resulting elastomer itself.

EP 0 332 038 A2

# TERT-ALKYL-PHENYLENEDIAMINES

## TECHNICAL FIELD

This invention pertains to mono and di-tert-alkyl derivatives of para-phenylenediamine.

## BACKGROUND OF THE INVENTION

Alkylated aromatic diamines have been known for a substantial period of time and find use in the preparation of polyurethane elastomers. The diamines are typically used in that form as chain extenders for polyurethanes, i.e., forming a short chain urea linkage to strengthen the elastomer. As is known, the alkyl group alters the reactivity of the amine thus giving the composition unique processing properties for producing polyurethane/polyurea elastomers.

A secondary use for the alkylated diamine products is in the manufacture of diisocyanates which are also suited for the synthesis of polyurethane elastomers. They can also be used for plasticizers, or as intermediates for the manufacture of pesticides and alkyd resin modifiers, and antioxidant. Represented patents showing alkylated aromatic diamines and use are as follows:

U.S. 4,482,690 discloses the manufacture and use of tert-butylbenzenediamine where the amine groups are meta to each other with the tert-butyl group ortho to an amine group and the use of the composition for chain extension in polyurethanes. The tert-butyl group reduces the reactivity of an amine group.

U.S. Patents 3,428,610 and 4,218,543 disclose the use of alkylated toluenediamines in the manufacture of polyurethane resins with the '543 patent showing its use in RIM manufacturing techniques. Alkylated diamines include 1-methyl-3,5-diethylphenylene-2,4-diamine and 1,3,5-trimethylphenylene-2,4-diamine. The diethyltoluenediamine derivative is referred to as diethyl TDA or DETDA and is probably the most widely used alkylated derivative of toluenediamine for RIM manufacture.

U.S. 2,762,845 shows 2,6-diethylaniline, isopropylaniline, ethyltoluidine and diethyl-m-toluidine.

British Patent 846,226 discloses both ortho and para-tert-butyl aniline;

U.S. 3,275,690 discloses isopropyl- and diisopropylaniline, mono and di-tert-butylaniline; ethylaniline and dimethyldiethylaniline.

AS 1,051,271 (West German) shows mono, di, and triisopropylaniline; and U.S. 3,222,401 discloses cycloalkyl derivates of aniline such as ortho-cyclooctylaniline, ortho and para-(dimethylcyclohexyl) aniline and ortho and para-methylcyclopentylaniline.

U.S. 4,440,952 shows the synthesis of 1-methyl-2,4-diamino-5-isopropylbenzene and 1-methyl-2,6-diamino-3-isopropylbenzene and the use of the 2,6-isomer as a chain extender for polyurethane formulation.

European patent 0069286 discloses various alkyl-substituted phenylenediamines as chain extenders for the production of polyurethanes by reaction injection molding techniques. Some of the compositions suggested as being suited for such use include 1,3-dimethyl-5-tert-butyl-2,6-diaminobenzene, 2-methyl-4,6-di-tert-butyl-1,3-diaminobenzene, and 1,3-dimethyl-5-tert-amyl-2,4-diaminobenzene.

European Patent 0107108 discloses the synthesis of $C_{1-4}$ alkylated vicinal toluenediamines and their use as extender for the preparation of polyurethane-polymer elastomers. The example showed an ethylated vicinal toluenediamine.

European Patent 177,916 discloses the preparation of mono- and di-tert-butyl-toluenediamine and other alkyl-substituted toluenediamines where the alkyl group is ortho to the amine and the use of such alkyl-substituted toluenediamines for the preparation of polyurethane polyureas.

## SUMMARY OF THE INVENTION

This invention pertains to mono and di-tert-alkyl-para-phenylenediamines. The compositions are best represented by the formulas:

I

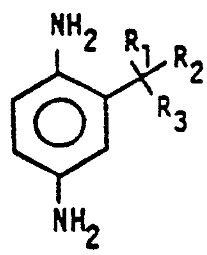

II

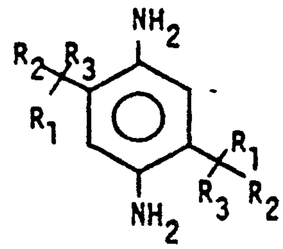

wherein $R_1$, $R_2$, and $R_3$ are $C_{1-3}$ alkyl groups or $R_2$ and $R_3$ are combined to form a $C_{5-6}$ membered ring.

There are several advantages associated with the specific para-phenylenediamine compounds of this invention. One advantage is that the mono and di-tert-alkyl-para-phenylenediamine compositions have a reactivity which is slower than para-phenylenediamine and many other alkylated para-phenylenediamines, thus making the compound more acceptable for the RIM urethane manufacture of a variety of large automobile parts. Second, mono-tert-alkylated-para-phenylenediamine is a low melting solid which is well suited for formulation handling; para-phenylenediamine is not. In addition mono-tert-alkyl-para-phenylenediamine has selective differential reactivity between the amine groups which provides for specialty applications. Third, these tert-alkylated-para-phenylenediamines impart excellent physical properties to urethane elastomers; e.g., tear strength, thermal stability and tensile strength.

## DETAILED DESCRIPTION OF THE INVENTION

The primary differences between the compositions described herein and those in the prior art, are: the compositions are mono and di-alkyl substituted para-phenylenediamine derivatives containing at least one tertiary alkyl group ortho to one amino group and the alkyl group contains a tertiary carbon atom, e.g. a tert-butyl radical. The prior art focused on meta-phenylene diamines and substitution thereof.

The compounds of this invention can be synthesized by alkylating para-phenylenediamine compounds. This permits substitution of the alkyl group ortho to the amine group. The art has utilized heterogeneous catalyst systems to effect alkylation of aromatic amines, eg., toluenediamines. Examples of these catalytic systems include silica-alumina, montmorillonite, crystalline molecular sieves and zeolites. Of these, highly acidic silica-alumina catalysts and crystalline zeolites and crystalline molecular sieves appear to offer advantages in that they have desired acidity and they can accommodate selective production of the mono-tert-alkyl-para-phenylenediamine product. Others may produce a mixture of mono and di-alkyl-substituted phenylenediamines. Crystalline zeolites are well known and basically are alumino-silicates where the mole ratio of silica to alumina is very high, e.g., greater than 3:1. Such zeolites cans be substituted with various cations to alter their acidity and thus their activity with respect to achieving alkylation of para-phenylenediamine. These cations can also alter the effective pore size of the zeolite and thus alter their catalytic activity. Two common types of zeolites are the X zeolite and the Y zeolite with the Y zeolite typically having a higher silica content. Typically a Y zeolite will have a silica to alumina molar ratio of about 3:1. Although the X and Y zeolites are common examples of zeolites suited for practicing the invention, others include faujasite, offretite, chabazite, K, L, omega, and the ZSM family. The Y zeolite or faujasite because of its silica to alumina ratio and pore configuration is well suited for the production of many mono-tert-alkyl-para-phenylenediamine products. This is particularly true with respect to the reaction of isobutylene with para-phenylenediamine to produce tert-butyl-para-phenylenediamine.

As noted, the acidity of zeolites can be controlled by utilizing various cations in place of the sodium atom in a synthetic or naturally occurring zeolite. The preferred cations suited for the catalyst system include the rare earth metals, such as, lanthanum, cerium, praseodymium, as well as various transition metals and hydrogen. Basic metal exchanged zeolites, which render the resulting zeolite weekly acidic, are relatively inactive. Alkali metals, in particular, decrease the reactivity of the exchanged zeolite. For this reason the highly or strongly acidic zeolites, i.e., hydrogen, rare earth metal exchange or those with a high silica to alumina ratio are preferred.

The pore size of zeolites has an influence on the reactivity and type of products produced. The pores may be altered toward either larger or smaller sizes by variety of known means. For example, it is known

that pore sizes may be reduced by chemical modification, e.g. addition of oxides of boron and phosphorus, or coke formation. It is also possible to render many zeolitic systems capable of synthesizing products having large molecular sizes by increasing the effective pore size of the zeolite from that in its unaltered state. The primary method of enlarging pore size is through removing alumina from the catalyst structure. Some of the main techniques for removing alumina are hydrothermal treatment (steaming) and acid washing. Thus, a zeolite which may have insufficient pore size to accommodate entry of the molecules for reaction may be expanded to have an effective pore size that will accommodate entry of such molecules. Alternatively, if the pore size is large substantial amounts of the di-alkyl-para-phenylenediamine products may be generated.

The mono and di-alkyl-para-phenylenediamines are prepared by reacting an olefin with para-phenylenediamine in the presence of an acidic catalyst. Examples of olefins for producing a para-phenylenediamine derivative having a tertiary-carbon atom include isobutylene, isoamylene, 2-ethyl-1-pentene, 2-ethyl-2-pentene, 2-methyl-1-pentene, 2-methyl-2-pentene, isooctene, and 1-methylcyclohexene. The relationship of the branched chain and olefin must be such that a tertiary carbon is available for alkylation. In this way $R_1$, $R_2$ and $R_3$ can have from 1 to 3 carbon atoms or $R_2$ and $R_3$ can be combined to form a 5 or 6 membered ring.

Typically, temperatures will range from about 100 to 250° C; preferably, 160 to 200° C and the pressures will range from about 15 to 2000 psig and generally in the range of 100 to 1000 psig. It is common practice to alter the temperature and pressure within the above ranges specified to optimize the selectivity and conversion to the desired product. Mole ratios of olefin to para-phenylenediamine used in the reaction will range from about 1 to 10:1 and the reaction time will generally be from about 2 to 48 hours. Where fixed bed catalytic reactors are used, the feed to the reactor, expressed as an LHSV, will range from about 0.05-6 hours $^{-1}$.

The following examples are provided to illustrate embodiments of the invention for synthesizing the tert-alkyl-para-phenylenediamines to illustrate performance of these phenylene diamines in the manufacture of polyurethane resins.

## Example 1

### Synthesis of 2-tert-butyl-1,4-phenylenediamine

A 30.0 g. portion of H-Y zeolite (powder) and 300 g. (2.78 mol) of para-phenylenediamine were charged to a 1000 cc Hastalloy C pressure vessel equipped with a mechanical stirrer. The vessel was sealed and purged with nitrogen, leaving 30 psig nitrogen blanket. The contents were heated to a designated temperature with stirring. Isobutylene (234 g., 4.18 mol) was then added over 15 minutes, resulting in an initial reaction pressure as indicated. The reaction mixture was maintained at that temperature for about 20 hours with constant stirring and then cooled to 150° C. Stirring was discontinued and the residual pressure was vented. Upon removal of the catalyst by hot filtration, a product mixture was obtained with then was separated by vacuum distillation. Conditions for four runs and product composition with conversion and selectivity are set forth as follows:

| Run | Temp. (° C) | Initial Pres. (psig) | % Conversion $Ph(NH_2)_2$ | % Product Selectivity of tert -butyl-phenylenediamines | | |
|---|---|---|---|---|---|---|
| | | | | N-t-Butyl | 2-t-Butyl | Σ di-t-Butyl |
| 1 | 180 | 821 | 66.99 | 50.26 | 49.73 | trace |
| 2 | 200 | 857 | 76.38 | 32.53 | 56.10 | 11.47 |
| 3 | 215 | 1,176 | 90.44 | 14.48 | 71.03 | 14.50 |
| 4 | 220 | 1,024 | 80.22 | 7.72 | 69.00 | 24.28 |

The reaction product mixture comprising N-tert-butyl-para-phenylenediamine, 2-tert-butyl-para-phenylenediamine and N, ring di-tert-butyl- and 2,5-di-tert-butyl-para-phenylenediamines was separated by fractional vaccum distillation. Azeotropes were broken by hydrocarbon addition. Product 2-tert-butyl-para-phenylenediamine was obtained as a low melting solid. The product then was recrystallized from hydrocarbon solvents with the exclusion of air to prevent oxidation. The product was analyzed by capillary GC/MS, and NMR. In addition, the melting point was determined to be 73.5-74.5° C.

## Example 2

### Synthesis of 2,5-di-tert-butyl-1,4-phenylenediamine

The procedure of Example 1 (run 4) was repeated to produce a reaction mixture comprising largely 2-t-butyl-para-phenylenediamine and di-t-butyl-para-phenylenediamine. The reaction mixture, after reaction was cooled and distilled to remove the 2-tert-butyl-para-phenylenediamine and other lights from the reaction medium. The distillation cut has the following analysis:

| | |
|---|---|
| 2-tert-butyl-para-phenylenediamine | 2.1% |
| 2,5-di-tert-butyl-para-phenylenediamine | 77.8% |
| Other di-tert-butyl-para-phenylenediamine | 17.7% |
| Others | 2.4% |

A 100 gram fraction of the distillation cut was added to 250 milliliters of toluene and heated to boiling to dissolve all solids. After all the solids were in solution, the solution was cooled to 25° C, filtered and washed with 100 milliliters of toluene. Approximately 75 grams of wet cake were recovered and that cake was recrystallized in 150 milliliters of toluene. After drying in an oven overnight, a slightly off-white solid was obtained, melting point above 150° C. Recrystallization to analytical purity resulted in a 170° C m.p.

## Example 3

### Chain Extender Reactivity

A series of polyurethane-urea elastomers utilizing various chain extenders was prepared and evaluated in a pot life test system for the purpose of determining the reactivity of the chain extenders in standard urethane formulations relative to one another. The polyurethane-urea elastomers were formulated by reacting a prepolymer of poly (1,4-oxytetramethylene) glycol end-capped with 2,4-toluenediisocyanate, the prepolymer having an NCO content generally from 5-7%, with the test candidate chain extender aromatic diamine and a diol. The nominal stoichiometric equivalent ratio of isocyanate to diamine chain extender to diol for each sample is 2:1:1. Commercially, the prepolymer is marketed under the trademark ADIPRENE L-167 by the E.I. duPont de Nemours Company and the poly($\epsilon$-caprolactone) diol marketed under the designation CAPA 200 by Interox Chemicals Limited.

The test system for measuring pot life comprised a heated test chamber for holding 7 grams of test sample at a constant temperature (50° C) and was equipped with a vertical perforated piston. This piston moves up and down through the test sample in timed relationship. (The temperature rise due to the exothermic reaction is discounted.) The force necessary to move the piston through the polymer sample is measured in arbitrary units, and the relationship of the force is plotted as a function of time. The force-pot life relationship of the urethane system during cure then is correlated to known force-viscosity relationships for the urethane-urea systems.

Table 2 represents tabular data providing coefficients for an empirical model expressing the logarithm base 10 of viscosity as a third-power polynomial function of time for several chain extender systems. The coefficients apply to the equation:

$$\log(\text{viscosity}) = I + A(\text{time}) + B(\text{time})^2 + C(\text{time})^3.$$

The coefficient of the first power term ("A") is a "quasi" reaction rate constant measuring initial reactivity.

Smaller values in the quasi rate constant (A) indicate longer and desirable pot lives. The value T/5000 is the time in which the reaction product has a relative viscosity of 5000 units. Even though the value is an arbitrary value, that value is relevant for use in determining the performance of the test candidate in a RIM process or cast elastomers process. For injection molding of modest size parts, T/5000 may be about 2.5 minutes; whereas, the molding of large or intricate parts may require a T/5000 of greater than 5 minutes, e.g., 10 to 40 minutes for hand casting.

Relative performance of tert-butyl-para-phenylenediamine candidates in comparison to other aromatic diamine chain extenders may be considered from Table I. In carrying out the potlife tests, some parameters were changed to compensate for the reactivity of the test candidates, namely, utilizing lower levels of chain extender. For example, in the test program, para-phenylenediamine reacted too fast for measurement in the urethane formulation at a normal 2/1/1 diisocyanate to diol to diamine chain extender ratio. The test was repeated using a mole ratio of 2/1.67/0.33 diisocyanate to diol to chain extender with para-phenylenediamine and with 2-cyclopent-2-enyl-para-phenylenediamine; the potlife time was 0.29 and 0.92 minutes, respectively. When tert-butyl-para-phenylenediamine was substituted in the 2/1.67/0.33 formulation, the reaction rate was too slow to cure within a reasonable time to a T/5000 viscosity. The formulation was adjusted to 2/1.33/0.67 mole ratio diisocyanate/diol/aromatic diamine chain extender and repeated. At that diisocyanate/diol/diamine ratio, the T/5000 of tert-butyl-para-phenylenediamine was accurately measurable as 4.08 minutes. To quantify the retardation of the 2-tert-butyl group to para-phenylenediamine as compared to the retardation of the tert-butyl group in meta-phenylenediamine as disclosed in U.S. 4,482,690, the intermediacy of 2-cyclopent-2-enyl-para-phenylenediamine was utilized. The rate quotient of 2-tert-butyl-para-phenylenediamine/2-cyclopent-2-enyl-para-phenylenediamine was (4.08/0.34) times the rate quotient of para-phenylenediamine/2-cyclopent-2-enyl-para-phenylenediamine (0.92/0.29). The calculated relative rate reduction imparted by the 2-tert-butyl group to paraphenylenediamine was 38 times and contrasted unexpectedly with the rate reduction imparted by the tert-butyl group to meta-phenylenediamine (10.74/1.1 or 9.8 times). Thus, the 2-tert-butyl group was approximately four times more effective in relative rate reduction vis-a-vis para-phenylenediamine as it was in rate reduction in meta-phenylenediamine.

TABLE 1

CHAIN EXTENDER REACTIVITY

Empirical Model of Potlife Data

log (relative viscosity) = I + A*(time) + B*(time)$^2$ + C*(time)$^3$

| acronym | T/5000 | A | B | C | I |
|---|---|---|---|---|---|
| mPD | 1.10 | 3.113 | -2.492 | .9681 | 1.958 |
| tBmPD | 10.7 | .1374 | -.0013 | -.00003 | 2.354 |
| .33 pPD | .29 | 8.087 | -2.156 | .1493 | 1.103 |
| .33 CppPD | .92 | 3.529 | -1.005 | .5066 | .8959 |
| .67 CppPD | .34 | 11.90 | -43.74 | 83.85 | 1.137 |
| .67 tBpPD | 4.08 | .6748 | 0.0570 | .0016 | 1.747 |
| tBpPD | 2.23 | 1.509 | -.3204 | .0337 | 1.522 |
| DtBpPD | 7.97 | .5234 | -.0381 | .0012 | 1.385 |

mPD = meta-phenylenediamine
tBmPD = 4-tert-butyl-meta-phenylenediamine
pPD = para-phenylenediamine
CppPD = 2-cyclopent-enyl-para-phenylenediamine
tBpPD = 2-tert-butyl-para-phenylenediamine
DtBpPD = 2,5-di-tert-butyl-para-phenylenediamine

* A Side:
B Side:

Example 4

## Cast Elastomers

Cast elastomers were prepared from the ortho-tert-butylated-para-phenylenediamine whose potlife T/5000 value was but 2.2 minutes. Conventional hand-mix techniques were used for these chain extenders, methylene bis(orthochloroaniline) (MOCA), chlorotoluenediamine and tert-butyl-metaphenylenediamine and 2,6-toluenediamine in combination with Adiprene-167 to prepare a series of molded test plaques whose physical properties were tested and are compared.

Specifically, the conventional toluenediisocyanate end-capped polytetramethyleneglycol was degassed at 90-100 °C under partial vacuum. When bubbling ceased the prepolymer was weighed and vigorously mixed with the proper weight of molten aromatic diamine chain extender to give the isocyanate index reported in the table. The resulting mixture was poured into an aluminum mold whose 150x150x1.9 mm cavity was properly pretreated with mold release agent.

After curing under pressure in a 30 ton hydraulic press at 100 °C for two hours, the test plaques were demolded, cured in an air oven at 100 °C for 22 hours, then postcured at ambient conditions for seven days before being conditioned for analytical testing by exposure to 23±2 °C at 50±5% relative humidity for 40 hours. Physical properties were measured in accordance with ASTM procedures. Hardness (ASTM D2240) and tensile (ASTM D1708) measurements are the average of five determination each, tear resistance (ASTM D624), die C) the average of three determinations.

Table 2 provides test data with respect to physical properties.

TABLE 2

| ADIPRENE 167 CAST POLYURETHANES | | | | | |
|---|---|---|---|---|---|
| Chain Extender | MOCA | Cl-TDA | tBmPD | 3tB26TDA | tBpPD |
| NCO Index | 1.05 | 1.04 | 1.05 | .95 | 1.05 |
| Shore A Hardness | 90 | 90 | 93 | 94 | 90 |
| Shore D Hardness | 47 | 39 | 40 | 44 | 40 |
| 100% tensile (psi) | 1680 | 1360 | 840 | 1520 | 1310 |
| 200% tensile (psi) | 2390 | 2080 | 1330 | 2380 | 2200 |
| 300% tensile | 3320 | 2940 | 2000 | 3440 | 3610 |
| Ratio 300/100 tensile | 1.98 | 2.16 | 2.38 | 2.26 | 2.76 |
| Break tensile (psi) | 4849 | 3050 | 5180 | 5490 | 5330 |
| % elongation (%) | 440 | 320 | 630 | 480 | 400 |
| Tear resistance (pli) | 770 | 530 | 490 | 660 | 570 |

MOCA = methylenebis(orthochloroaniline)
Cl-TDA = chlorotoluenediamine
tBmPD = 4-tert-butyl-meta-phenylenediamine
3tB26TDA = 3-tert-butyl-2,6-toluenediamine
tBpPD = 2-tert-butyl-para-phenylenediamine

These data show that the tensile strength and 300 °/100% tensile ratios for the tert-butylpara-phenylenediamine system are superior to any of the other chain extenders except 3tB26TDA. It should also be noted the isomer tBmPD resulted in poorer overall elastomer properties than tBpPD not only in the 300/100 tensile ratios, but in tear resistance. Thus, even though tBmPD is less reactive than tBpPD in terms of pot life, it does not provide the excellent physical properties achieved by tBpPD.

## Example 5

## RIM ELASTOMER PREPARATION

Reaction injection molded elastomers were prepared using a model SA8-20 laboratory machine (LIM Kunststoff Technologie Gmbh, Kittsee, Austria) suitable for processing two component mixtures. 10-30 cc/min metering pumps for components "A" (methylenediphenyldiisocyanate, MDI) and "B" (polyol plus chain extender plus catalysts) are driven synchronously by sprocket wheels in proportion to the mixture to be processed by means of a variable speed (50-250 rpm) motor. Any desired mixing ratio may be established by changing gears. Components A and B are conveyed into a mixing chamber by individually controlled compressed air actuated valves. A high speed rotor, continuously adjustable from 10,000 to 18,000 rpm using a frequency transformer, mixes the components. The pump block and mixing head are movable, and may be automatically advanced to a stationary mold by compressed air. A replaceable 'O' ring accomplishes a seal between mixing the head and mold.

In a first test series polyurethane-urea elastomers from commercial modified liquid methylene diphenyldiisocyanate (MDI) reaction with moderate molecular weight (1000-3000 equivalent wt) triols supplemented with test phenylene diamine chain extender was made. An isocyanate index of 1.05 was sought for all elastomers and checked by machine 'calibration shots' of unmixed, unreacted A and B components through the model SA8-20 sampling ports designed for this purpose. Stream temperatures were set by controlled circulation of thermostatted water through the double walled A and B reservoirs, the mixing block temperature by electrical resistance heaters. Molds were thermostatted before mounting on a jig to which the mixing head was conveyed during the injection molding operation. 200x200x2 and 200x200x3 mm cavities in nominally 26x27x4 cm aluminum molds were treated with mold release agents before each injection. After injection the mixing rotor was washed in situ with dioctylphthalate, blown clean with nitrogen and readied for the next injection shot as the mold was unmounted and opened. Test plaques were cured for one hour at 250 °C and properly conditioned for replicate hardness, tensile and tear tests on 2 mm thick pieces as noted above for cast elastomers. The additional variable of yield tensile is reported for the crosslinked RIM elastomers due to the characteristic shape of the Instron stress-strain curve. Also tabulated are flexural modulus and maximum stress (ASTM D1708) determined on each of five 1"x3" specimens from the 3 mm plaques and sag, a measure of thermal stability (ASTM D3769) measured on a 100 mm overhang using 3 mm thick plaque samples. Mold fillability was determined at a set mold fill rate. A filled mold contains about 140-150 g of elastomer.

Tables 3 and 4 set forth the results. Multranol PF is a long chain triol obtained from Mobay Corporation and Mondur PF is an isocyanate obtained from Mobay Corporation. The term OPD refers to ortho-phenylenediamine, mPD refers to meta-phenylenediamine and pPD refers to para-phenylenediamine. The test results for this first series are shown in Table 3.

TABLE 3

| RIM REACTIVITY COMPARISON | | | |
|---|---|---|---|
| Chain Extender | oPD | mPD | pPD |
| Chain Extender PPH | | 6.7 | |
| Polyol | | Multranol 3901 | |
| Isocyanate | | Mondur PF | |
| NCO Index | | 1.03 | |
| Shore A | 80 | 72 | 85 |
| Shore D | 32 | 27 | 31 |
| 100 °C Tensile (psi) | 720 | 500 | 660 |
| Break Tensile (psi) | 1840 | 1450 | 920 |
| % Elongation | 440 | 310 | 240 |
| Tear Strength (psi) | 370 | 230 | 230 |
| Mold fill (gm) | 142 | 140 | 7.2 |

Table 3 shows the remarkably poor precessibility of para-phenylenediamine. It is poorly soluble in polyol and at only 6.7 parts para-phenylenediamine per 100 parts polyol a mold fill of less than 5% was achieved. Cognates ortho-phenylenediamine or meta-phenylenediamine resulted in mold fill to the 140-150 gm level.

In a second test series the tert-butyl counterparts of the first test series were evaluated in elastomer formation. The test series was also compared with a mix of tert-butyl-toluenediamine (tBTDA) where the 2,4-/2,6-isomer was in the ratio of 80/20 parts by weight. The results are set forth in Table 4.

TABLE 4

| RIM URETHANE FORMULATION | | | |
|---|---|---|---|
| CHAIN EXTENDER | tBpPDA | tBTDA | tBoTDA |
| CH_EX pph | 20.3 | 22 | 22 |
| POLYOL | | VORANOL 4815 | |
| ISOCYANATE | | ISONATE 181 | |
| NCO index | 1.05 | 1.05 | 1.05 |
| Catalyst_1 | 33-LV | 33-LV | 33-LV |
| Cat_1 pph | .063 | .063 | .063 |
| Catalyst_2 | UL-28 | UL-28 | UL-28 |
| Cat_2 pph | .063 | .063 | .063 |
| A Stream temp °C | 39.9 | 41.4 | 42.9 |
| B Stream temp °C | 39.3 | 45.6 | 41.0 |
| Mixing head temp °C | 39.6 | 39.9 | 39.9 |
| Shore A Hardness | 95 | 96 | 96 |
| Shore D Hardness | 54 | 56 | 55 |
| 100% tensile (psi) | 2270 | 2030 | 1260 |
| 200% tensile (psi) | 3230 | 2740 | 1680 |
| 300% tensile (psi) | - | | 2220 |
| yield tensile (psi) | 1620 | 2020 | 1310 |
| break tensile (psi) | 3740 | 3240 | 3440 |
| % elongation (%) | 270 | 270 | 490 |
| tear resistance (pli) | 790 | 690 | 590 |
| maximum stress (psi) | 1520 | 1880 | 1330 |
| flexural modulus (psi) | 30500 | 40000 | 25300 |
| sag (250°F, 1 hr) (4") | 0.156 | 0.33 | 0.72 |
| sag (250°F, 1 hr) (6") | 1.13 | 1.85 | - |

Glossary

tBpPDA = 2-tert-butyl-para-phenylenediamine
tBoTDA = tert-butyl-ortho-toluenediamine
Voranol 4815 = Dow polyether polyol
Isonate 181 = Upjohn methylenediphenyldiisocyanate
tBTDA = tert-butyl-toluenediamine (80% 2,4- and 20% 2,6-toluenediamine isomer ratio)
33-LV = Air Products and Chemicals, Inc. triethylene diamine in dipropylene glycol catalyst
UL-28 = Witco Tin Catalyst
pph = weight parts chain extender or catalyst per hundred parts polyol

Table 4 presents the current composition benefit of good polyol solubility and RIM processability for 2-tert-butyl-para-phenylenediamine. The RIM elastomer data show the improved thermal stability of the tert-butyl-para-phenylenediamine at the same number of equivalents as tert-butyl-meta or ortho-toluenediamines. The data show that tear resistance and tensile at break was excellent for the tert-butyl-para-phenylenediamine chain extended system. Also there is significantly lower sag even at lower chain extender levels thus showing enhanced thermal stability of the elastomer as compared to tBTDA.

Example 6

The procedure of Example 5 was repeated for test candidates diethyltoluenediamine, tert-butyl-toluenediamine (80/20 mixture as in Example 5 and tert-butyl-meta-phenylenediamine). The formulation was modified utilizing the Example 5 Mondur PF isocyanate at an NCO index of 1.03 with Multranol 3901 polyol and an amine catalyst system of UL-28 and triethylenediamine at 0.132 parts per 100 parts Multranol 3901. This example also differs from Example 5 in that the chain extender level was increased to approximately 29.5 for the tert-butyl-metaphenylenediamine as compared to 32 parts for both the tert-butyl-toluenediamine and diethyltoluenediamine. Table 5 sets forth the results.

TABLE 5

| HIGH MODULUS RIM CHAIN EXTENDER | | | |
|---|---|---|---|
| Chain Extender | DETDA | tBTDA | tBmPD |
| Chain Extender (pph) | 32.0 | 32.0 | 29.5 |
| A/B Gears | 60/46 | 60/46 | 57/45 |
| A/B Ratio | .5904 | .5919 | .6050 |
| PLAQUE #s tested | 35-AB | 36-AB | 37-AB |
| Shore A Hardness | 97 | 98 | 97 |
| Shore D Hardness | 66 | 71 | 68 |
| 100% tensile (psi) | 3590 | 3150 | 2890 |
| 200% tensile (psi) | 4630 | 3940 | 3750 |
| 300% tensile (psi) | . | 4890 | . |
| Yield tensile (psi) | 3050 | 3150 | 2940 |
| Break tensile (psi) | 5030 | 4820 | 4330 |
| Elongation (%) | 240 | 300 | 260 |
| Tear resistance (pli) | 920 | 910 | 860 |
| Maximum stress (psi) | 3190 | 3800 | 3320 |
| Flexural modulus (psi) | 65320 | 79300 | 70700 |
| Sag (250°F, 1 hr, "/4") | .043 | .081 | .133 |
| Sag (250°F, 1 hr, "6/") | .350 | .396 | .535 |
| Mold Fill (g) | 101 | 108 | 148(Full) |
| * A Side:<br>B Side: | | | |

From Table 5 it is noted that both tert-butyl-toluenediamine as a mixture and diethyltoluenediamine (also an 80/30 mixture) were faster reacting than tBmPD. However, in terms of elastomer properties in this High Modulus RIM Chain Extender formulation both the DETDA and tBTDA produce substantially superior elastomers to tBmPD in terms of break tensile, tear resistance, flexural modulus and thermal stability as evidenced by the 250°F sag. The elastomer containing the tBmPD chain extender exhibited substantially more sag than tBTDA and DETDA. In contrast to the results in the urethane formulation set forth in Table 4, the thermal stability of a urethane formulation utilizing tBpPDA was less than an elastomer formulated with tBTDA. From these data it is clear that superior properties are afforded in elastomers, in terms of tensile strength, tear resistance, and thermal stability utilizing tert-butyl-paraphenylenediamine as the chain extender as compared to tert-butyl-meta-phenylenediamine.

## Claims

1. A 1,4-phenylenediamine composition having an alkyl substituent in the 2-position or 2 and 4-position and having a tertiary carbon atom represented by the formulas:

I

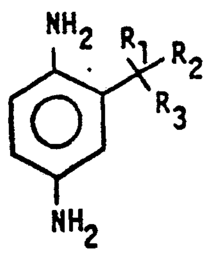

II

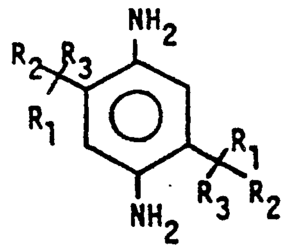

wherein $R_1$, $R_2$, and $R_3$ are $C_{1-3}$ alkyl groups or $R_2$ and $R_3$ are combined to form $C_{5-6}$ membered ring.

2. The composition of Claim 1 which is represented by formula I.

3. The composition of Claim 2 wherein $R_1$, $R_2$, and $R_3$ are $C_1$ methyl groups.

4. The composition of Claim 2 wherein $R_1$ is ethyl, $R_2$ is a $C_1$ methyl and $R_3$ is a $C_1$ methyl group.

5. The composition of Claim 2 wherein $R_1$ is $C_1$ methyl and $R_2$ and $R_3$ are combined into a ring having from 5-6 carbon atoms.

6. The composition of Claim 2 wherein $R_1$ is $C_2$ ethyl, $R_2$ is $C_2$ ethyl and $R_3$ is $C_1$ methyl.

7. The composition of Claim 2, wherein $R_1$ is $C_1$ methyl, $R_2$ and $R_3$ are combined and represent a six carbon membered ring.

8. The composition of Claim 1 represented by formula II.

9. The composition of Claim 8 wherein $R_1$ is $C_1$ methyl and $R_2$ and $R_3$ are $C_1$ methyl or $C_2$ ethyl groups.

10. The composition of Claim 8 wherein $R_1$ is $C_1$ methyl and $R_2$ and $R_3$ are combined to form a 5 carbon membered ring.

11. The composition of Claim 8 wherein $R_1$ is $C_2$ ethyl, $R_2$ and $R_3$ are $C_1$ methyl groups.

12. The composition of Claim 8 wherein $R_1$ is $C_1$ methyl and $R_2$ and $R_3$ are combined into a ring having 6 carbon atoms.

13. The composition 2-tert-butyl-1,4-diaminobenzene.